# EUROPEAN PATENT SPECIFICATION

(11) **EP 1 865 944 B1**
(45) Date of publication and mention of the grant of the patent: **29.08.2018**
(21) Application number: 06739849.5
(22) Date of filing: 28.03.2006
(51) Int. Cl.: A61K 31/198, A61K 31/405, A61K 31/4172, A61P 3/00, A61P 3/02, A61P 3/06, A61P 3/10, A61P 5/50, A61P 19/08, A61P 21/00, A23L 33/175

(54) **Amino acid-containing composition used for preventing or remedying decrease in skeletal muscle mass of aged people, comprising L-leucine**
Aminosäure-enthaltende Zusammensetzung zur Vorbeugung oder Behandlung der Abnahme der Skelettmuskelmasse von älteren Menschen, enthaltend L-Leucin
Composition à base d'acides aminés utilisée pour prévenir ou remédier à la diminution de la masse musculaire squelettique de personnes âgées, comprenant de la L-leucine

(30) Priority: 29.03.2005 US 665849 P
(43) Date of publication of application: 19.12.2007
(73) Proprietor: Ajinomoto Co., Inc., Tokyo 104-8315 (JP)
(72) Inventor: KOBAYASHI, Hisamine AJINOMOTO CO., INC., Kawasaki-shi, Kanagawa (JP); SUZUKI, Hiromi AJINOMOTO CO., INC., Kawasaki-shi, Kanagawa (JP); WOLFE, Robert, Little Rock, AR 72205 (US); KATSANOS, Christos THE UNIVERSITY OF TEXAS MEDICAL, Galveston, Texas (US)
(74) Representative: Dunne, Paul David
(86) International application number: PCT/US2006/011325
(87) International publication number: WO 2006/105112

(56) References cited:
- WO-A1-2004/019928
- WO-A1-2004/026294
- WO-A1-2007/049818
- WO-A2-2005/034932
- US-A- 5 789 443
- US-A1- 2003 187 049
- KATSANOS CHRISTOS S ET AL: "Effects of a leucine-enriched amino acid drink on muscle protein synthesis in the elderly", FASEB JOURNAL, FED. OF AMERICAN SOC. FOR EXPERIMENTAL BIOLOGY, US, vol. 19, no. 5, Suppl. S, Part 2, 6 March 2005 (2005-03-06), page A1573, XP009155490, ISSN: 0892-6638
- DATABASE WPI Week 2003 Thomson Scientific, London, GB; AN 2003-819102 XP002667569, "Agent useful in health food/beverage products, food adjuvants and pharmaceuticals for preventing and treating abnormal glucose tolerance and hepatopathy, comprises leucine, isoleucine and/or valine as active ingredient", -& JP 2003 171271 A (AJINOMOTO KK) 17 June 2003 (2003-06-17)
- VOLPI ELENA ET AL: "Essential amino acids are primarily responsible for the amino acid stimulation of muscle protein anabolism in healthy elderly adults", THE AMERICAN JOURNAL OF CLINICAL NUTRITION, AMERICAN SOCIETY FOR NUTRITION, US, vol. 78, no. 2, 1 August 2003 (2003-08-01) , pages 250-258, XP002609729, ISSN: 0002-9165
- KATSANOS C S ET AL: "A high proportion of leucine is required for optimal stimulation of the rate of muscle protein synthesis by essential amino acids in the elderly", AMERICAN JOURNAL OF PHYSIOLOGY - ENDOCRINOLOGY AND METABOLISM 2006 US LNKD- DOI:10.1152/AJPENDO.00488.2005, vol. 291, no. 2, 28 February 2006 (2006-02-28), pages E381-E387, XP002667571, ISSN: 0193-1849

## Description

### Technical Field

The present invention relates to an amino acid-containing composition which is orally ingested for preventing or remedying a decrease in the skeletal muscle of aged people.

### Background Art

An age-related decrease of muscle mass occurs in skeletal muscle, which is called sarcopenia. The decrease of skeletal muscle mass lowers the muscle strength and function, which becomes a big reason why aged people come to require nursing care. Since a decrease in the skeletal muscle mass is also a cause of metabolism-related diseases such as decrease of insulin-sensitivity, diabetes mellitus and osteoporosis, it is becoming a medical and social problem in this super-aging society.

A decrease in skeletal muscle mass takes place when muscle protein synthesis rate becomes less than its degradation rate. Therefore, the countermeasure is to promote the protein anabolism of skeletal muscle by increasing the muscle protein synthesis or reducing muscle protein degradation.

In order to improve the protein metabolism of skeletal muscle and promote the protein anabolism, administration of a hormone preparation, etc. to aged people has been attempted, which produces side effects (see, S. E. Borst, Age and Ageing, 2004, 33:548-555).

In attempts at trophotherapy, when a multiple nutritional supplement was ingested by aged people, no improvement in cross-sectional area of thigh muscle was observed (see, M. A. Fiatarone, et al., The New England Journal of Medicine, 1994, 330:1769-1775). At that time, however, intake of nutrition from ordinary food decreases as a result of ingestion of the nutritional supplement, and amount of nutrition intake as a whole decreased rather than increasing. Thus, a nutritional supplementary food which does not disturb the intake of ordinary nutrition is necessary.

While the aged people took a physical exercise for 12 weeks, muscle hypertrophy took place when a nutritional supplementary food containing 10 g of protein, 7 g of carbohydrate and 3 g of lipid was ingested immediately after the physical exercise (see, B. Esmarck, et al., Journal of Physiology, 2001, 535:301-311). However, when the nutritional supplementary food was ingested 2 hours after the physical exercise, no muscle hypertrophy took place. There has been a demand for a nutrition supplementary food that can prevent the muscle from becoming atrophied, independently of physical exercise.

Amino acids are materials for the synthesis of protein and a protein anabolism of amino acids has been well known. It has been reported that, even in aged people, synthesis of muscle protein is enhanced and anabolism of muscle protein is promoted when amino acids are ingested *per os* (see, E. Volpi, et al., Journal of Clinical Investigation, 1998, 101:2000-2007).

It has been also reported that, when 18 g of a mixture of essential amino acids was ingested by aged people, protein synthesis of skeletal muscle increased. Thus, it has been confirmed that, in an amino acid mixture which is orally ingested by aged people intended for promotion of muscle protein anabolism in aged people, only the essential amino acids are sufficient. (see, E. Volpi, et al., American Journal of Clinical Nutrition, 2003, 78:250-258). However, ingestion of a large amount of amino acids, as high as 18 grams at a time, to aged people is not practical for aged people, so that amino acid mixtures having more efficacy are needed.

Further, there is also a report on the experiment where a composition containing nine essential amino acids and two non-essential amino acids, lipid, and carbohydrate was administered to aged people having type II diabetes mellitus in a dosage of 16.58 g over a period of 16 weeks. It is reported that there was no change in their body weights although improvements were observed in the blood sugar level, hemoglobin A_{1c} (HbA_{1c}), insulin concentration and the like (see, S.B. Solerte, et al., American Journal of Cardiology, 2004, 93:23A-29A).

With regard to essential amino acids, it has been known that a nutritional composition in which each essential amino acid is contained in a specific ratio produces a good absorption, whereby utilization rate of nitrogen is high, so that such a nutritional composition is an effective nutrient in treating various kinds of diseases (see, US 5,132,113 A).

Among amino acids, leucine has been known to control the protein anabolism (see, K. S. Nair, et al., American Journal of Physiology Endocrinology and Metabolism, 1992, 263:E928-E934). However, it has been also reported that, in order to maintain the synthesis of body protein, not only leucine but also all amino acids are necessary (see, M. Frexes-Steed, et al., American Journal of Physiology, Endocrinology and Metabolism, 1992, 262:E925-E935). In light of this, many amino acid intravenous solutions in which the ratio of branched amino acids including leucine is increased have been put on the market. However, such commercially available solutions are not designed for routine oral administration for aged people, but used when oral administration of nutrients is impossible due to injury, disease, surgery or the like.

It is reported that, when a solution which was prepared by adding leucine to a commercially available amino acid intravenous solution so that the concentration of leucine is 35% based on total amount of nitrogen (mole ratio of leucine is 69% with respect to total of essential amino acids) was intravenously administered to scald rabbits under general anesthesia over five hours, such a solution promoted the synthesis of muscle protein in comparison with a commercially available amino acid intravenous solution (mole ratio of leucine is 17% with respect to total of essential amino acids) or a solution which was prepared by adding leucine to a commercially available amino acid intravenous solution so that the concentration of leucine is 25% based on total amount of nitrogen (mole ratio of leucine is 58% with respect to total of essential amino acids) (see, X-J. Zhang, et al., Journal of Nutrition, 2004, 134:3313-3318). However, such a solution is not also designed for routine oral administration for aged people. Further, an orally ingested amino acid-containing composition having high efficacy is not known.

Various other documents propose the use of leucine-containing compositions for treating medical conditions:
WO 2004/026294 discloses a composition comprising leucine and at least one of isoleucine, lysine, methionine, phenylalanine, threonine, tryptophan, valine, or histidine in free and/or salt form, wherein leucine in free and/or salt form is present in an amount of at least about 10 to about 35% by weight based on the weight of total amino acids. The compositions are said to be suitable for promotion of muscle protein synthesis or controlling tumor-induced weight loss.

Katsanos et al. disclose an amino acid drink comprising whey protein based essential amino acids enriched with leucine. The drink is said to have a stimulatory effect on muscle protein synthesis in older individuals (see FASEB Journal, Fed. Of American Soc. For Experimental Biology, US, vol. 19, no. 5, Suppl. S, Part 2, 6 March 2005).

JP 2003/171271 discloses medicines for treating hepatopathy, abnormal glucose tolerance and diabetes, having at least one amino acid selected from leucine, isoleucine and valine.

WO 2004/019928 discloses therapeutic agents for liver disease, which comprises isoleucine, valine and alanine as active ingredients.

As mentioned above, there has been an increasing demand for amino acid mixtures that can be orally ingested in a continuous manner without disturbing the normal ingestion of nutrition and that can effectively improve skeletal muscle protein metabolism independently of physical exercise.

### Disclosure of the Invention

An object of the present invention is to provide an amino acid-containing nutritional composition which can be easily taken by aged people and which is capable of improving metabolism of skeletal muscle to effectively prevent the decrease of muscle protein or promote the accumulation of the muscle protein even when the intake of the composition is not accompanied with physical exercise.

As a result of earnest studies for solving the above-mentioned problems, the present inventors have found that an amino acid-containing composition where L-leucine is contained at a particular amount ratio in total of essential amino acids can well improve the metabolism of the skeletal muscle protein of aged people to prevent or remedy a decrease in the skeletal muscle protein of aged people. In addition, the above-mentioned amino acid-containing composition has been found to improve insulin-sensitivity, diabetes and impaired carbohydrate metabolism of aged people, or hyperlipemia, fatty liver and impaired lipid metabolism of aged people. The present invention has been thus achieved.

Namely, the present invention is as follows:
(1) An amino acid composition orally administered for use in preventing or remedying age-related decrease in skeletal muscle mass of aged people, wherein each of amino acids is contained at the following molar composition ratio (%) with respect to total of essential amino acids: 0.0 to 3.5 of L-histidine, 5.0 to 15 of L-isoleucine, 35 to 45 of L-leucine, 12 to 16 of L-lysine, 3.0 to 10 of L-methionine, 3.5 to 8.0 of L-phenylalanine, 11 to 14 of L-threonine, 8.5 to 15 of L-valine, 0.0 to 1.0 of L-tryptophan, and 0.0 to 10 of L-arginine.
(2) An amino acid composition orally administered for use according to (1) which is a pharmaceutical product, nutrient or supplement.
(3) An amino acid composition orally administered for use according to (1) or (2) which is also for use in preventing, or remedying, deterioration of insulin-sensitivity, diabetes, or a disorder of carbohydrate metabolism.
(4) An amino acid composition orally administered for use according (1) or (2) which is also for preventing or remedying hyperlipidemia, fatty liver or impaired lipid metabolism.

The protein anabolism of skeletal muscle can be promoted to prevent or remedy a decrease of the skeletal muscle mass of aged people by oral administration of the amino acid-containing composition comprising L-leucine at a specific ratio of the present invention to aged people.

Also, oral administration of the above-mentioned composition to aged people can prevent or remedy abnormal carbohydrate metabolism such as deterioration of insulin-sensitivity, diabetes or the like which may be accompanied by the decrease of skeletal muscle mass of aged people.

Further, oral administration of the above-mentioned composition to aged people can prevent or remedy abnormal lipid metabolism such as increased lipid concentration in the blood or liver which may be accompanied by the decrease of skeletal muscle mass of aged people.

### Brief Description of Drawings

Fig. 1 shows changes of fractional synthesis rate of skeletal muscle protein.
Fig. 2 shows amount of phenylalanine incorporated into lower limb as an index for accumulated mass of skeletal muscle protein. There is a significant difference between two groups.
Fig. 3 shows increases in lean body mass (LBM), an index of muscle mass, from baseline (the start of amino acid supplementation). **p*<0.05 vs. baseline.
Fig. 4 shows increases in leg muscle strength from baseline (the start of amino acid supplementation). **p*<0.05 vs. baseline.
Fig. 5 shows changes in blood glucose level during oral glucose tolerance test.
Fig. 6 shows changes in blood insulin level during oral glucose tolerance test.
Fig. 7 shows changes in blood cholesterol level from baseline (the start of amino acid supplementation). **p*<0.05 vs. baseline.
Fig. 8 shows changes in blood triglyceride level from baseline (the start of amino acid supplementation). **p*<0.05 vs. baseline.
Fig. 9 shows changes in blood very low-density lipoprotein (VLDL) level from baseline (the start of amino acid supplementation). **p*<0.05 vs. baseline.
Fig. 10 shows changes in liver fat. **p*<0.05 vs. week 0 (baseline).
Fig. 11 shows fractional synthesis rates (FSR) of skeletal muscle protein. **p*<0.05 vs. Leu/EAA 25%.
Fig. 12 shows fractional synthesis rates (FSR) of skeletal muscle protein. **p*<0.05 vs. Leu/EAA 30%.
Fig. 13 shows fractional synthesis rates of skeletal muscle protein.
Fig. 14 shows changes of blood glucose level during oral glucose tolerance test.
Fig. 15 shows hemoglobin A1c (HbA1c). There is a significant difference between two groups.
Fig. 16 shows liver fat contents. There is a significant difference between two groups.

### Best Mode for Carrying Out the Invention

The term "aged people" herein used means people aged 60 and over.

The term "essential amino acids" herein used include nine kinds: L-histidine, L-isoleucine, L-leucine, L-lysine, L-methionine, L-phenylalanine, L-threonine, L-valine, and L-tryptophan.

The amino acid-containing composition of the present invention contains 35 to 45% of L-leucine in terms of molar composition ratio with respect to total of essential amino acids.

The respective molar composition ratios (%) with respect to total of essential amino acids are: 0.0 to 3.5 of L-histidine, 5.0 to 15 of L-isoleucine, 35 to 45 of L-leucine, 12 to 16 of L-lysine, 3.0 to 10 of L-methionine, 3.5 to 8.0 of L-phenylalanine, 11 to 14 of L-threonine, 8.5 to 15 of L-valine, and 0.0 to 1.0 of L-tryptophan. A physiologically acceptable salt or hydrates of each amino acid also can be used. Examples are L-lysine hydrochloride and L-histidine hydrochloride monohydrate.

The amino acid-containing composition of the present invention may be either in the form of a free amino acid, peptide or protein, or mixture thereof. The indispensable condition is that the total content of L-leucine is 35% or more in terms of molar ratio.

It is appropriate that the amino acid-containing composition of the present invention is ingested every day. The amount to be ingested is preferably about 3 to 11 g per ingestion, and about 7 to 22 g a day.

The amino acid-containing composition of the present invention may further comprise an additive. For example, other nutrients such as saccharides, lipids, proteins, vitamins, minerals and the like may be incorporated alone or in combination into the composition. In this case, the composition may further comprise an additive such as an excipient, flavoring agent, coloring agent or the like. The amino acid-containing composition of the present invention thus manufactured may be put on the market as it is or in the form of powder or liquid mixture, or after processed into a jelly or food product.

The amino acid-containing composition of the present invention, which is designed for oral injection for aged people, is provided in the form of a food product, pharmaceutical product, nutrient or supplement.

### [Examples]

### Example 1

An essential amino acid mixture comprising the amino acid constitution of whey protein as shown in Table 1 was orally ingested by ten aged people (3 females and 7 males, average age 66.7 year, average body weight 81.7 kg) while a high-leucine essential amino acid composition where the proportion of leucine was enhanced as shown in the same Table 1 were orally ingested by ten other aged people (5 females and 5 males, average age 66.5 year, average body weight 74.5 kg). The total amount of ingested amino acids was 6.726 grams in both groups. L-Phenylalanine which was labeled with a stable isotope was used and the fractional synthesis rate of muscle protein was measured for 3.5 hours after ingestion of the amino acids, and the change from the fractional synthesis rate measured before ingestion of amino acid was computed. Moreover, the difference in concentrations in blood of phenylalanine between femoral artery and femoral vein and the blood flow rate were measured and the net amount of phenylalanine incorporated in the lower limb 3.5 hours after ingestion of amino acids was determined as an index for accumulated mass of muscle protein.

**[Table 1] Amino Acid Composition (molar ratio to total of essential amino acids).**

| | Essential Amino Acids of Whey Protein Composition * | High-Leucine Essential Amino Acids |
|---|---|---|
| L-Histidine | 3.91% | 3.06% |
| L-Isoleucine | 11.88% | 9.30% |
| L-Leucine | 26.17% | 42.22% |
| L-Lysine | 18.56% | 14.52% |
| L-Methionine | 4.83% | 3.78% |
| L-Phenylalanine | 6.11% | 4.78% |
| L-Threonine | 15.99% | 12.51% |
| L-Valine | 12.56% | 9.83% |

| | | |
|---|---|---|
| *Comparative composition | | |

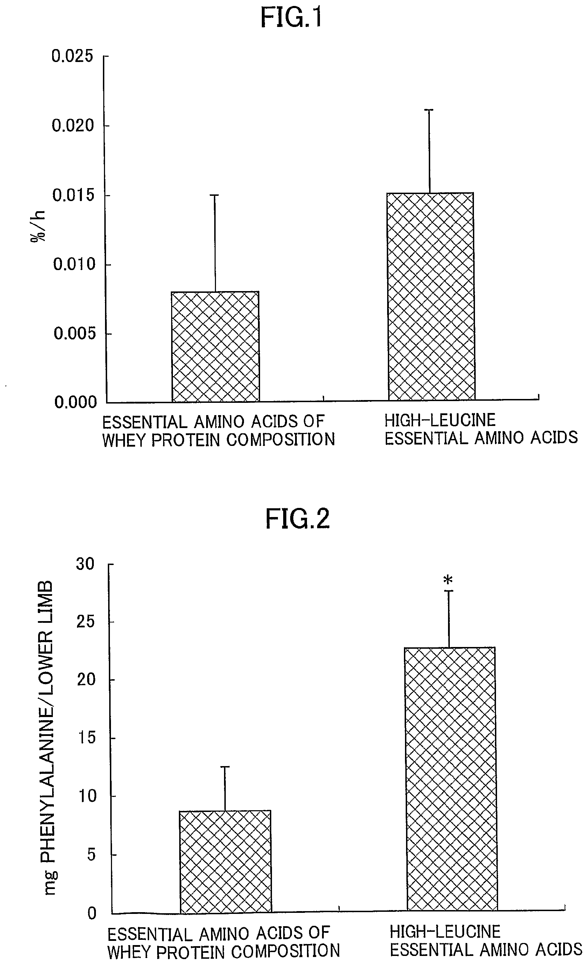

Fig. 1 shows changes in fractional synthesis rates (FSR) of muscle protein by amino acid ingestion. In the group which ingested the high-leucine composition of essential amino acids, an increase of the fractional synthesis rate of muscle protein was greater than that of the group which ingested the essential amino acid composition of whey protein.

Fig. 2 shows amount of phenylalanine incorporated into the lower limb. In the group which ingested the high-leucine composition of essential amino acids, there was a significant increase in the amount of phenylalanine incorporated into the lower limb or, in other words, an increase in accumulated amount of muscle protein, compared with the group which ingested the essential amino acid constitution of whey protein. The above results indicate that a high-leucine essential amino acid composition has a very good effect on promotion of anabolism of muscle protein in aged people.

### Example 2

Eight aged people with insulin resistance (4 females and 4 males, average age 66.1 year, average body weight 80.1 kg) was supplemented with an amino acid mixture, shown in Table 2, for sixteen weeks. The amino acid mixture was orally ingested as two daily doses of 11 grams between meals. Lean body mass (LBM) was measured as an index of muscle mass by dual energy X-ray absorptiometry (DEXA) before the start of amino acids supplementation and every four weeks. Leg muscle strength was determined as a sum of one repetition max (1-RM) of knee flexors and knee extensors. 75 grams oral glucose tolerance test (OGTT) was conducted to assess insulin sensitivity and blood lipid concentrations were determined every four weeks. Liver fat was determined by magnetic resonance imaging (MRI) every eight weeks.

**[Table 2] Amino Acid Composition (molar ratio to total of essential amino acids, L-Arginine is considered a nonessential amino acid).**

| | |
|---|---|
| L-Histidine | 3.23% |
| L-Isoleucine | 10.06% |
| L-Leucine | 42.12% |
| L-Lysine | 14.40% |
| L-Methionine | 3.70% |
| L-Phenylalanine | 4.34% |
| L-Threonine | 12.37% |
| L-Valine | 9.78% |
| L-Arginine | 8.81% |

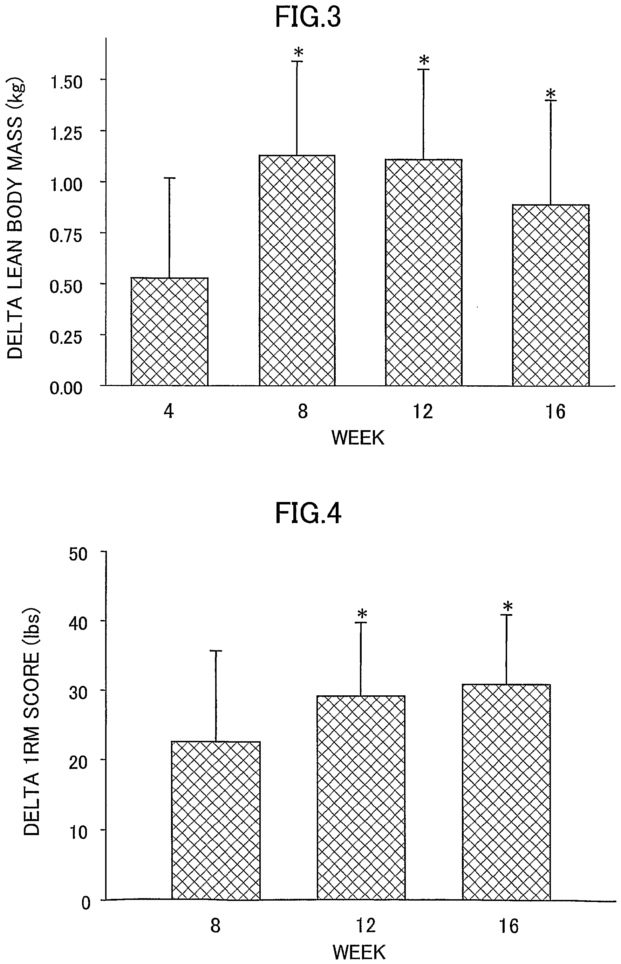

Fig. 3 shows increases in lean body mass (LBM), an index of muscle mass, from the start of amino acid supplementation. The average lean body mass before the start of amino acid supplementation was 51.54 kg. At eight weeks of amino acid supplementation or later, lean body mass was markedly increased.

Fig. 4 shows increases in leg strength from the start of amino acid supplementation. The average leg strength before the start of amino acid supplementation was 314.11 lbs. At twelve weeks of amino acid supplementation or later, leg strength was markedly increased.

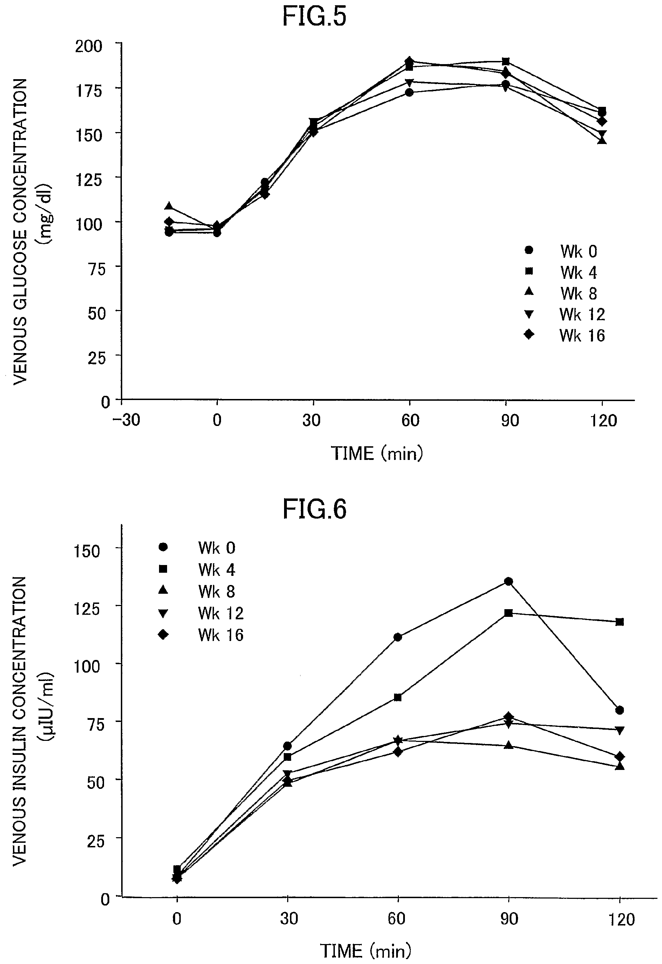

Fig. 5 shows changes in blood glucose level during oral glucose tolerance test. The amino acid supplementation did not affect blood glucose level.

Fig. 6 shows changes in blood insulin level during oral glucose tolerance test. At eight weeks of amino acid supplementation or later, blood insulin level was lowered. Since blood glucose level during oral glucose tolerance test was unchanged (Fig. 5), it is suggested that insulin sensitivity was improved by the amino acid supplementation.

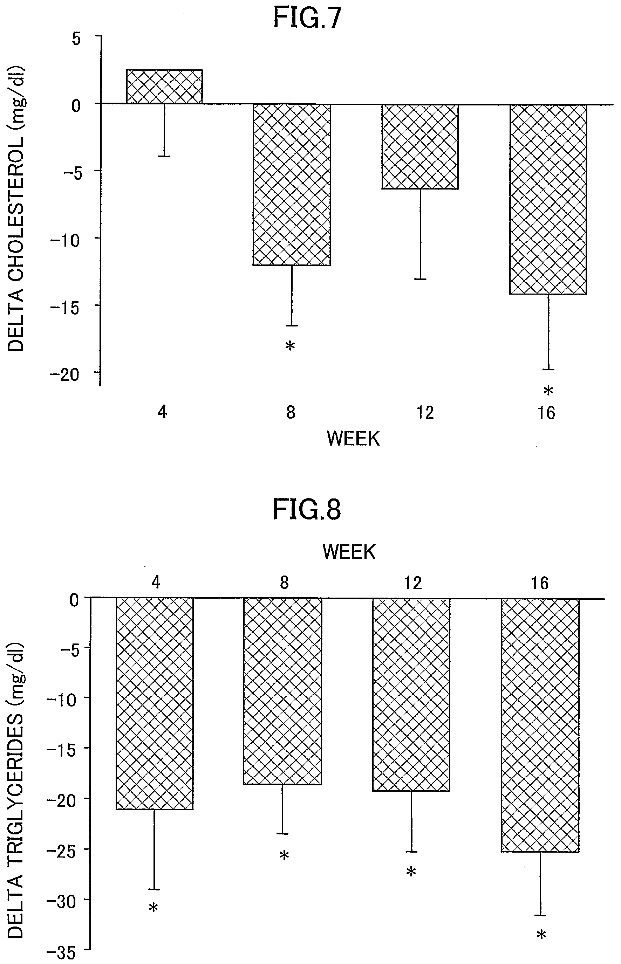

Fig. 7 shows changes in blood cholesterol level. The average blood cholesterol level before the start of amino acid supplementation was 191 mg/dl. At eight and sixteen weeks of the amino acid supplementation, blood cholesterol level was markedly decreased.

Fig. 8 shows changes in blood triglyceride level. The average blood triglyceride level before the start of amino acid supplementation was 117 mg/dl. Blood triglyceride level was markedly decreased by the amino acid supplementation.

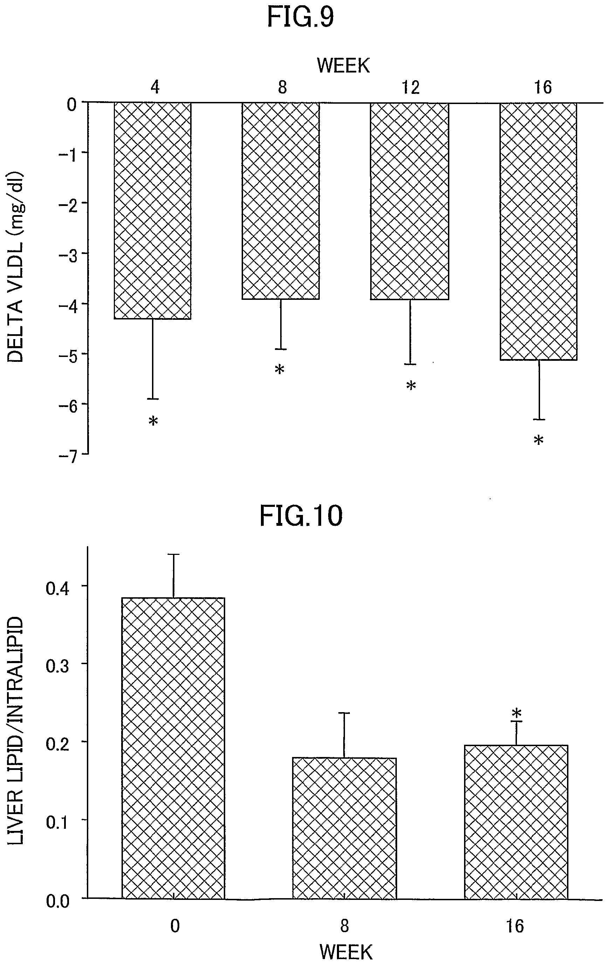

Fig. 9 shows changes in blood very low-density lipoprotein (VLDL) level. The average blood VLDL level before the start of amino acid supplementation was 23 mg/dl. Blood VLDL level was markedly decreased by the amino acid supplementation.

Fig. 10 shows changes in liver fat. It is observed that amino acid supplementation reduced liver fat.

### Example 3

Male SD strain rats (ten weeks old) were orally administrated with 3.5 % amino acids aqueous solutions, compositions are shown in table 3, at a dose of 20 ml/kg (dose of amino acids was 0.7 g/kg). The compositions of amino acids were comprised based on whey protein. To be more concrete, a total amino acid mixture contains 25% L-leucine as a molar ratio to total essential amino acids, which is an amino acid composition of whey protein (Leu/EAA 25% TAA), an essential amino acid mixture contains 49% L-leucine as a molar ratio to total essential amino acids (Leu/EAA 49% EAA), a total amino acid mixture contains 58% L-leucine as a molar ratio to total essential amino acids (Leu/EAA 58% TAA), or a total amino acid mixture contains 66% L-leucine as a molar ratio to total essential amino acids (Leu/EAA 66% TAA) were administered. Fractional synthesis rates of muscle protein were determined for 20 minutes between 30 and 50 minutes after the administration by flooding dose method using stable isotope labeled phenylalanine.

**[Table 3] Amino Acid Composition (molar ratio to total of essential amino acids).**

| | Leu/EAA 25% TAA* | Leu/EAA 49% EAA * | Leu/EAA 58% TAA* | Leu/EAA 66% TAA* |
|---|---|---|---|---|
| L-Histidine | 3.8% | 0.0% | 2.1% | 1.7% |
| L-Isoleucine | 11.6% | 8.7% | 6.5% | 5.3% |
| L-Leucine | 25.5% | 49.0% | 58.3% | 65.9% |
| L-Lysine | 18.0% | 13.5% | 10.1% | 8.2% |
| L-Methionine | 4.7% | 3.5% | 2.6% | 2.1% |
| L-Phenylalanine | 5.9% | 4.5% | 3.3% | 2.7% |
| L-Threonine | 15.5% | 11.7% | 8.7% | 7.1% |
| L-Tryptophan | 2.7% | 0.0% | 1.5% | 1.3% |
| L-Valine | 12.2% | 9.2% | 6.8% | 5.6% |
| Nonessential amino acids | 119.1% | 0.0% | 66.6% | 54.4% |

| | | | | |
|---|---|---|---|---|
| *Comparative compositions | | | | |

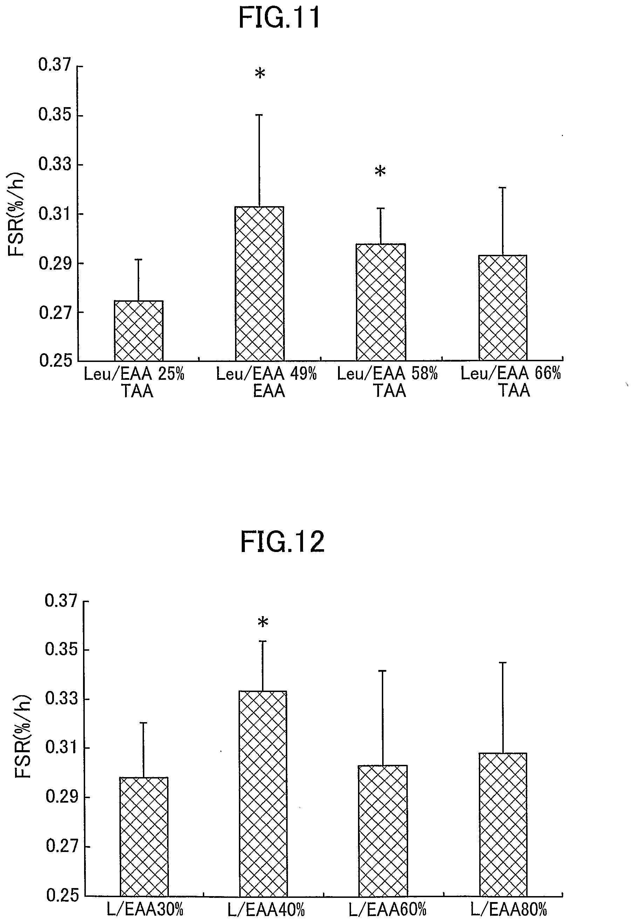

Fig. 11 shows fractional synthesis rates (FSR) of skeletal muscle protein. Fractional synthesis rates of muscle protein were grater in the groups administered with amino acid mixtures contains 49%, 58.3%, and 65.9% L-leucine as a molar ratio to total of essential amino acids than in the group administered with whey protein amino acid composition, which contains 25.5% L-leucine. Among them, fractional synthesis rate of muscle protein was significantly increased when 49% or 58.9% L-leucine containing amino acid mixture was administered.

### Example 4

Male SD strain rats (ten weeks old) were orally administrated with 3.5 % amino acids aqueous solutions, compositions are shown in table 4, at a dose of 20 ml/kg (dose of amino acids was 0.7 g/kg). The compositions of amino acids were comprised based on whey protein and designed as the doses of L-leucine were equal in all groups. Fractional synthesis rates of muscle protein were determined for 20 minutes between 30 and 50 minutes after the administration by flooding dose method using stable isotope labeled phenylalanine.

**[Table 4] Amino Acid Composition (molar ratio to total of essential amino acids).**

| | Leu/EAA 30%* | Leu/EAA 40% | Leu/EAA 60%* | Leu/EAA 80%* |
|---|---|---|---|---|
| L-Histidine | 3.6% | 3.1% | 2.0% | 1.0% |
| L-Isoleucine | 10.8% | 9.3% | 6.2% | 3.1% |
| L-Leucine | 30.0% | 40.0% | 60.0% | 80.0% |
| L-Lysine | 16.9% | 14.5% | 9.7% | 4.8% |
| L-Methionine | 4.4% | 3.8% | 2.5% | 1.3% |
| L-Phenylalanine | 5.6% | 4.8% | 3.2% | 1.6% |
| L-Threonine | 14.6% | 12.5% | 8.3% | 4.2% |
| L-Tryptophan | 2.6% | 2.2% | 1.5% | 0.7% |
| L-Valine | 11.5% | 9.8% | 6.6% | 3.3% |
| Nonessential amino acids | 0.0% | 33.3% | 100.0% | 166.7% |

| | | | | |
|---|---|---|---|---|
| *Comparative compositions | | | | |

Fig. 12 shows fractional synthesis rates of muscle protein. The fractional synthesis rate of muscle protein was most increased in the group of 40% L-leucine to total of essential amino acids, when the doses of L-leucine in all groups were fixed.

### Example 5

Male SD strain rats (seven weeks old) were orally administrated with 3.5% aqueous solutions of whey protein, a mixture of 50% whey protein and 50% amino acid (an amino acid composition of the mixture is shown in table 5), or an amino acid mixture which composition is same as the mixture of whey protein and amino acid shown in table 5, at a dose of 20 ml/kg (0.7 g/kg as amino acids). Fractional synthesis rates of muscle protein were determined for 20 minutes between 30 and 50 minutes after the administration by flooding dose method using stable isotope labeled phenylalanine.

**[Table 5] Amino Acid Composition (molar ratio to total of essential amino acids).**

| | |
|---|---|
| L-Histidine | 1.2% |
| L-Isoleucine | 10.6% |
| L-Leucine | 42.3% |
| L-Lysine | 14.7% |
| L-Methionine | 3.4% |
| L-Phenylalanine | 4.4% |
| L-Threonine | 11.9% |
| L-Tryptophan | 0.9% |
| L-Valine | 10.6% |
| Nonessential amino acids | 36.4% |

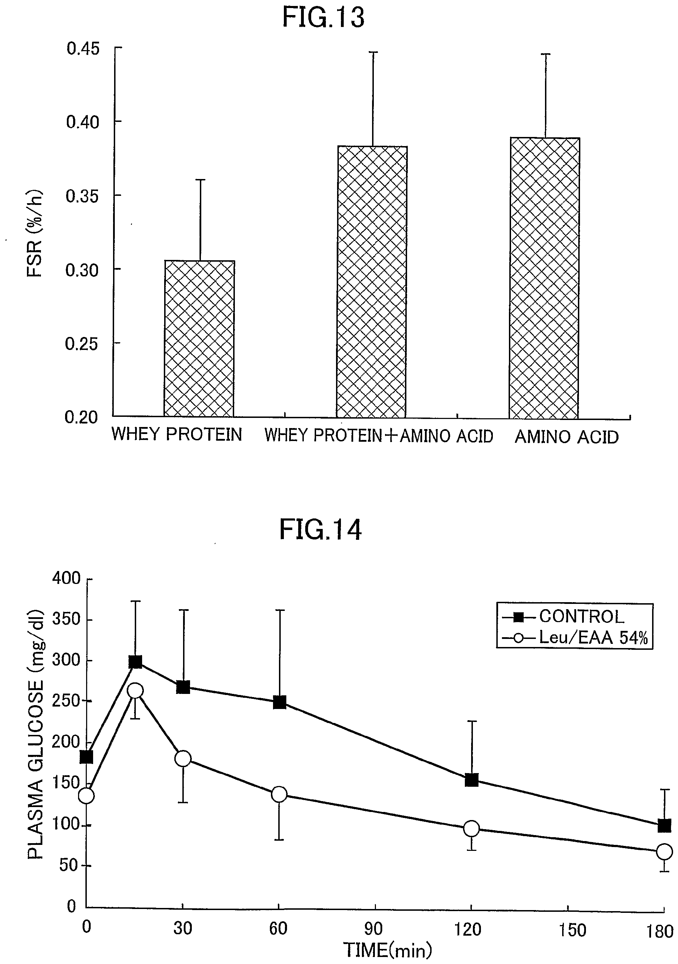

Fig. 13 shows fractional synthesis rates of muscle protein. The fractional synthesis rates of muscle protein were grater in the groups of the mixture of whey protein plus amino acids and the mixture of amino acids, both contain 42.3% L-leucine to total of essential amino acids, than in the whey protein group. The effects of the mixture of whey protein and amino acids and the mixture of amino acids were even.

Five weeks old male KK-Ay mice, an animal model of type 2 diabetes, were fed 25% high-fat diet for two weeks. After two weeks, they were fed 14% fat 12 % casein diet (Control) or "Control" diet supplemented with 8% amino acid mixture (Leu/EAA 54%), which composition was shown in table 6, contains 53.5% L-leucine to total of essential amino acids, for four weeks, followed by 1 g/kg BW oral glucose tolerance test. And hemoglobin A1c (HbA1c) and liver fat were determined.

**[Table 6] Amino Acid Composition (molar ratio to total of essential amino acids, L-Arginine is considered a nonessential amino acid).**

| | Leu/EAA 54%* |
|---|---|
| L-Histidine | 2.6% |
| L-Isoleucine | 8.1% |
| L-Leucine | 53.5% |
| L-Lysine | 11.6% |
| L-Methionine | 3.0% |
| L-Phenylalanine | 3.5% |
| L-Threonine | 9.9% |
| L-Tryptophan | 0.0% |
| L-Valine | 7.9% |
| L-Arainine | 7.1% |

| | |
|---|---|
| *Comparative composition | |

Fig. 14 shows changes of blood glucose level during oral glucose tolerance test. The blood glucose level was lowered in the group supplemented with amino acids, which contains 53.5% L-leucine to total of essential amino acids. And it is suggested that glucose tolerance was improved by the amino acid supplementation.

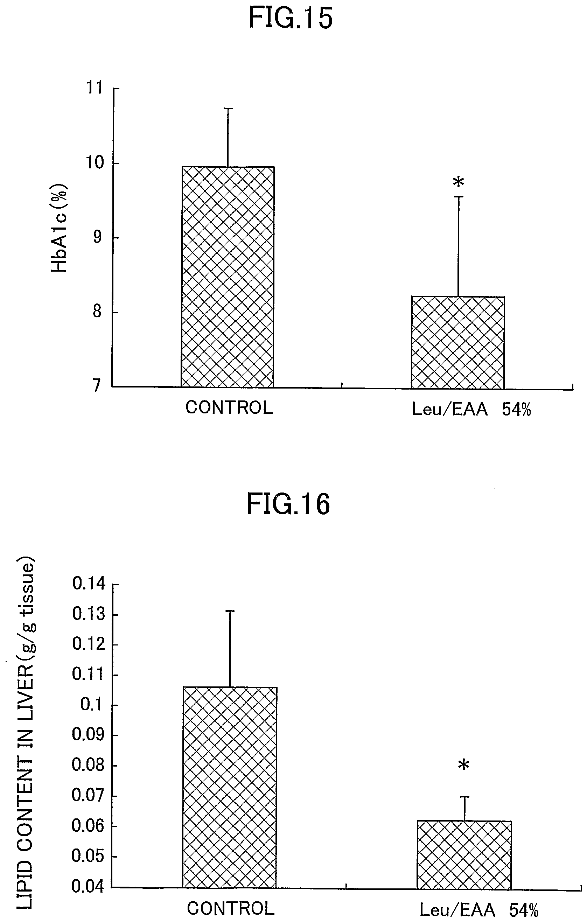

Fig. 15 shows hemoglobin A1c (HbA1c) which represents past blood glucose level. HbAlc was significantly lowered in the group supplemented with amino acids, which contains 53.5% L-leucine to total of essential amino acids. And it is suggested that blood glucose level was maintained lower during the experimental period by the amino acid supplementation.

Fig. 16 shows liver fat contents. Liver fat was significantly decreased in the group supplemented with amino acids, which contains 53.5% L-leucine to total of essential amino acids. And it is suggested that lipid metabolism was improved by the amino acid supplementation.

## Claims

1. An amino acid composition orally administered for use in preventing or remedying age-related decrease in skeletal muscle mass of aged people, wherein each of amino acids is contained at the following molar composition ratio (%) with respect to total of essential amino acids: 0.0 to 3.5 of L-histidine, 5.0 to 15 of L-isoleucine, 35 to 45 of L-leucine, 12 to 16 of L-lysine, 3.0 to 10 of L-methionine, 3.5 to 8.0 of L-phenylalanine, 11 to 14 of L-threonine, 8.5 to 15 of L-valine, 0.0 to 1.0 of L-tryptophan, and 0.0 to 10 of L-arginine.

2. An amino acid composition orally administered for use according to claim 1 which is a pharmaceutical product, nutrient or supplement.

3. An amino acid composition orally administered for use according to claim 1 or 2 which is also for use in preventing, or remedying, deterioration of insulin-sensitivity, diabetes, or a disorder of carbohydrate metabolism.

4. An amino acid composition orally administered for use according to claim 1 or 2 which is also for preventing or remedying hyperlipidemia, fatty liver or impaired lipid metabolism.

## Patentansprüche

1. Aminosäurezusammensetzung, die oral verabreicht wird, zur Verwendung bei der Verhinderung oder Heilung einer altersbedingten Abnahme der Skelettmuskelmasse älterer Menschen, wobei jede der Aminosäuren im folgenden molaren Zusammensetzungsverhältnis (%) in Bezug auf die Gesamtheit der essentiellen Aminosäuren enthalten ist: 0,0 bis 3,5 von L-Histidin, 5,0 bis 15 von L-Isoleucin, 35 bis 45 von L-Leucin, 12 bis 16 von L-Lysin, 3,0 bis 10 von L-Methionin, 3,5 bis 8,0 von L-Phenylalanin, 11 bis 14 von L-Threonin, 8,5 bis 15 von L-Valin, 0,0 bis 1,0 von L-Tryptophan und 0,0 bis 10 von L-Arginin.

2. Aminosäurezusammensetzung, die oral verabreicht wird, zur Verwendung nach Anspruch 1, die ein pharmazeutisches/r Produkt, Nährstoff oder Nahrungsergänzungsmittel ist.

3. Aminosäurezusammensetzung, die oral verabreicht wird, zur Verwendung nach Anspruch 1 oder 2, die auch zur Verwendung bei der Verhinderung oder Heilung einer Verschlechterung von Insulinsensitivität, Diabetes oder einer Störung des Kohlenhydratstoffwechsels dient.

4. Aminosäurezusammensetzung, die oral verabreicht wird, zur Verwendung nach Anspruch 1 oder 2, die auch zur Verhinderung oder Heilung von Hyperlipidämie, Fettleber oder eingeschränktem Fettstoffwechsel dient.

## Revendications

1. Composition d'acides aminés administrée par voie orale destinée à prévenir ou à réduire la diminution liée au vieillissement de la masse musculaire squelettique des personnes âgées, dans laquelle chaque acide aminé est contenu au rapport de composition molaire suivant (%) par rapport au total d'acides aminés essentiels : 0,0 à 3,5 de L-histidine, 5,0 à 15 de L-isoleucine, 35 à 45 de L-leucine, 12 à 16 de L-lysine, 3,0 à 10 de L-méthionine, 3,5 à 8,0 de L-phénylalanine, 11 à 14 de L-thréonine, 8,5 à 15 de L-valine, 0,0 à 1,0 de L-tryptophane et 0,0 à 10 de L-arginine.

2. Composition d'acides aminés administrée par voie orale pour une utilisation selon la revendication 1, qui est un produit pharmaceutique, un nutriment ou un supplément.

3. Composition d'acides aminés administrée par voie orale pour une utilisation selon la revendication 1 ou 2, qui est également destinée à prévenir ou à remédier à la détérioration de la sensibilité à l'insuline, au diabète ou à un trouble du métabolisme des hydrates de carbone.

4. Composition d'acides aminés administrée par voie orale pour une utilisation selon la revendication 1 ou 2, qui est également destinée à prévenir ou à remédier à l'hyperlipidémie, la stéatose hépatique ou au métabolisme lipidique altéré.
